(19) **Europäisches Patentamt**

**European Patent Office**

**Office européen des brevets**

(11) **EP 1 384 484 B1**

(12) **EUROPEAN PATENT SPECIFICATION**

(45) Date of publication and mention of the grant of the patent:
**14.09.2005 Bulletin 2005/37**

(51) Int Cl.[7]: **A61K 38/10**, A61P 35/00, A61P 9/10

(21) Application number: **02016402.6**

(22) Date of filing: **22.07.2002**

(54) **Use of efrapeptin oligopeptides for inhibition of angiogenesis**

Verwendung von Efrapeptin Oligopeptiden zur Hemmung der Angiogenese

Utilisation doligopeptides efrapeptine pour inhiber l'angiogénèse

(84) Designated Contracting States:
**AT BE BG CH CY CZ DE DK EE ES FI FR GB GR IE IT LI LU MC NL PT SE SK TR**

(43) Date of publication of application:
**28.01.2004 Bulletin 2004/05**

(73) Proprietor: **Papathanasiou, Antonia**
**Thessaloniki 54622 (GR)**

(72) Inventor: **Papathanasiou, Antonia**
**Thessaloniki 54622 (GR)**

(56) References cited:
- **GUPTA S. ET AL.: "Structure of efrapeptins from the fungus Tolypocladium niveum : peptide inhibitors of mitochondrial ATPase." J. ORG. CHEM., vol. 57, 1992, pages 2306-2313, XP002225332**
- **BANDANI A.R. ET AL.: "Production of efrapeptins by Tolypocladium species and evaluation of their insecticidal and antimicrobial properties." MYCOLOGICAL RESEARCH, vol. 104, no. 5, May 2000 (2000-05), pages 537-544, XP009002638**
- **NAGARAJ G. ET AL.: "Antimalarial activities of peptide antibiotics isolated from fungi." ANTIMICROBIAL AGENTS AND CHEMOTHERAPY., vol. 45, no. 1, January 2001 (2001-01), pages 145-149, XP002225333**
- **MUROI M. ET AL.: "Efrapeptins block exocytic but not endocytic trafficking of proteins." BIOCHEM. BIOPHYS. RES. COMM., vol. 227, 1996, pages 800-809, XP002225334**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The invention relates to the use of efrapeptin oligopeptides for the treatment of angiogenesis-based diseases such as cancer, atherosclerosis, psoriasis, and macular degeneration.

Angiogenesis is the formation of new blood vessels from pre-existing ones through migration and proliferation of endothelial cells (Folkman, J., Angiogenesis and angiogenesis inhibition: an overview, *EXS.,* **79**, 1-8 (1997)).

With the exception of would healing and menstruation, angiogenesis in adults is observed only in pathological situations such as cancer, atherosclerosis, psoriasis, and macular degeneration, where it contributes to the progression and symptom manifestation of the disease. For example, it has been observed that solid tumors neither grow beyond a minimal size nor metastasize unless they become vascularized (Folkman, J., Angiogenesis in cancer, vascular, rheumatoid and other disease, *Nat. Med.* **1(1)**, 27-31 (1995) and Folkman, J., What is the Evidence that Tumors are Angiogenesis Dependent? *J. Natl. Canc. Inst.,* **82**, 4-6 (1990)).

The family of angiogenesis-related diseases also includes endometriosis, Kaposi's sarcoma and other HIV-related conditions, leukemia, myocardial angiogenesis, neovascular glaucoma, and diabetic retinopathy. As used herein, the term "angiogenesis-related diseases" means pathological conditions that require endothelial cell proliferation for progression and symptom manifestation (Chappey et al., Endothelial cells in culture: an experimental model for the study of vascular dysfunctions, *Cell Biol. Toxicol.,* **12** (**4-6**), 199-205 (1996)).

Increasing experimental evidence suggest that inhibition of angiogenesis in humans and animals suffering from an angiogenesis-related disease can lead to symptom alleviation, disease halt and/or cure. For example, compounds known to inhibit endothelial cell proliferation have been shown to inhibit tumor angiogenesis and to prevent tumor growth and metastasis in a variety of cancer animal models and in human subjects participating in human clinical trials (Eatock et al. Tumor vasculature as a target for anticancer therapy, *Cancer Treat. Rev.,* **26(3)**, 191-201 (2000)).

It is clear that, for the treatment of cancer and other angiogenesis-related diseases, what is needed is a method, which inhibits undesirable endothelial cell proliferation.

In accordance with the present invention, methods are provided for the treatment of diseases mediated by endothelial cell proliferation; these methods comprise administering to the human or animal a composition containing therapeutic dosages of efrapeptin oligopeptides. Efrapeptins are a family of apolar, hydrophobic peptides isolated from entomopathogenic fungi and composed of a common amino acids alanine, glycine, leucine and uncommon amino acids $\alpha$-aminobutyric acid, $\beta$-alanine, isovaline, and pipecolic acid with the amino-terminal acetylated and the carboxyl-terminal blocked by N-peptido-1-isobutyl-2-[1-pyrrole-(1,2-$\alpha$)-pyrimidinium,2,3,4,5,6,7,8-hexahydro]-ethylamine (Krasnoff et al. Antifungal and Insecticidal Properties of the Efrapeptins: Metabolites of the Fungus Tolypocladium niveum, *J. Invert. Path.,* **58**, 180-188 (1991)).

According to the present invention, efrapeptins show a remarkable suppression of growth factor-induced endothelial cell growth. Furthermore, administration of efrapeptins in a pharmaceutically suitable carrier inhibits the progression of angiogenesis-related diseases such primary tumor growth.

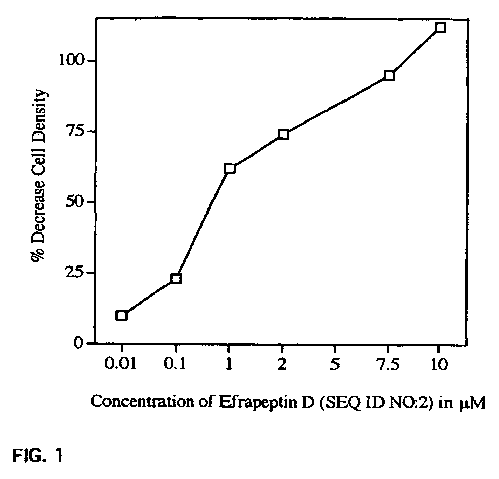

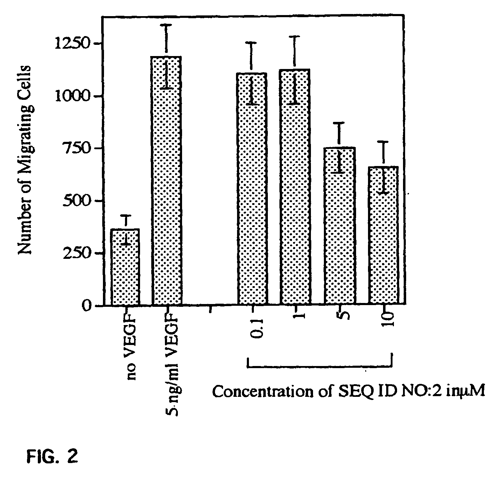

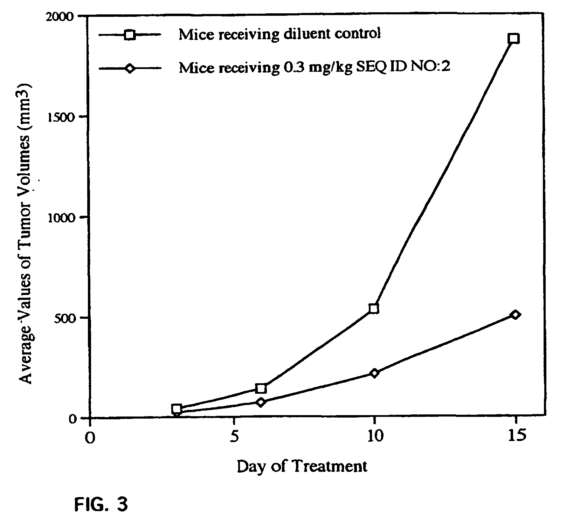

Fig. 1 shows inhibition of endothelial cell proliferation by efrapeptin D (SEQ ID NO:2). Fig. 2 shows inhibition of endothelial cell migration by efrapeptin D (SEQ ID NO:2). Fig. 3 shows inhibition of tumor growth after administration of efrapeptin D (SEQ ID NO:2).

Objects, features, and aspects of the present invention are disclosed in or are obvious from the following Detailed Description.

The present invention provides methods for the treatment of angiogenesis-related diseases. The treatment comprises the administration of an efrapeptin or efrapeptin mixture in sufficient amount to inhibit undesirable endothelial cell proliferation, particularly angiogenesisis, and most particularly angiogenesis-related diseases.

Example 1

*Effect of efrapeptin D (SEQ ID NO:2) on human endothelial cell growth.*

Human umbilical vein endothelial cells (HUVECs) were routinely cultured to confluency. The cells were then trypsinized, plated in a 96-well plate at 5,000 cells/well, and allowed to adhere for a period of time. Subsequently, the cells were stimulated to proliferate in the presence of 20 ng/ml basic fibroblast growth factor (bFGF). Concentrations of efrapeptin D (SEQ ID NO:2) ranging from 10 nM to 10$\mu$M were also added to the wells. After an incubation period of 48 hours, endothelial cell proliferation was determined using Cyquant Cell Proliferation Assay kit according to the manufacturer's instructions. Inhibition of cell proliferation is expressed as % Decrease Cell Density and calculated using the following formula:

$$\% \text{ Decrease Cell Density} = \frac{[\text{Fluorescence of bFGF-treated cells}]-[\text{Fluorescence of bFGF- and efrapeptin-treated cells}]}{[\text{Fluorescence of bFGF-treated cells}]- [\text{Fluorescence of untreated cells}]} \times 100$$

Efrapeptin D (SEQ ID NO:2) inhibited endothelial cell proliferation with an $IC_{50}$ of 0.75 µM. The relative antiproliferative effect of efrapeptin D (SEQ ID NO:2) is shown in Fig. 1.

Example 2

*Effect of efrapeptin D (SEQ ID NO:2) on migration of human endothelial cells.*

Migration of HUVECs was evaluated in modified Boyden chambers using a 48-well format consisting of upper and lower wells separated by a 8-micron pore polycarbonate filter coated with collagen. HUVECs were added to the lower wells and the migrating factor (5ng/ml of vascular endothelial growth factor or VEGF) was added to the upper wells. HUVECs were allowed to migrate from the lower to the upper wells for six hours at 37°C, 5%$CO_2$ in the presence and absence of concentrations of efrapeptin D (SEQ ID NO:2) ranging from 100 nm to 10 µM. At the end of the incubation period, the filters were removed and the cells on the filters were fixed, stained with hematoxylin, and counted with a light microscope.

Efrapeptin D (SEQ ID NO:2) inhibited VEGF-induced migration of HUVECs. The antimigratory effect of the efrapeptin oligopeptide is illustrated in Fig. 2. Values shown in Fig. 2 represent the mean often fields, bars represent standard deviation.

Example 3

*Antitumor effect of efrapeptin D (SEQ ID NO:2).*

Two groups of C57BL/6 mice numbering 9 mice/group were injected s.c. with $1 \times 10^6$ cells/0.2 ml Lewis Lung Carcinoma (LLC) cells. Treatment, which was initiated when the tumors became palpable, comprised of daily i.p. administrations of 0.3 mg/kg efrapeptin D (SEQ ID NO:2) or equal volume of diluent control. The animals received a total of fourteen treatments. Tumor volumes were recorded twice a week and assessed according to the formula: tumor volume=length x width$^2$ x $\pi$/6.

Administration of efrapeptin D (SEQ ID NO:2) significantly suppressed LLC tumor growth as illustrated in Fig.3.

### Claims

1. A use of an efrapeptin oligopeptide in the manufacture of a medicament for the treatment of diseases requiring the inhibition of angiogenesis.

2. The use of Claim 1 wherein the efrapeptin oligopeptide is selected from the group comprising SEQ ID NO:1, SEQ ID NO:2, SEQ ID NO:3, SEQ ID NO:4, and SEQ ID NO:5.

3. The use of Claim 2 wherein a disease related to excessive/uncontrolled angiogenesis is a disease selected from the group consisting of cancer, endometriosis, Kaposi's sarcoma, arthritis, atherosclerosis, psoriasis, macular degeneration, diabetic retinopathy, neovascular glaucoma, myocardial angiogenesis, and obesity.

### Patentansprüche

1. Die Anwendung eines Efrapeptin-Oligopeptids für die Herstellung eines Medikamentes zur Therapie von Krankheiten, bei denen die Inhibition der Angiogenese erforderlich ist.

2. Die Anwendung nach Anspruch 1, bei welcher das Efrapeptin-Oligopeptid aus der Gruppe bestehend aus SEQ ID NO: 1, SEQ ID NO: 2, SEQ ID NO: 3, SEQ ID NO: 4 und SEQ ID NO: 5 ausgewählt wird.

3. Die Anwendung nach Anspruch 2, bei welcher die Krankheit, die in Zusammenhang mit exzessiver/unkontrollierter Angiogenese steht, eine Krankheit ist, die aus der Gruppe bestehend aus Krebs, Endometriose, Kaposi-Sarkom, Arthritis, Atherosklerose, Psoriasis, Makuladegeneration, diabetischer Retinopathie, neovaskulärem Glaukom, myokardialer Angiogenese und Adipositas ausgewählt wird.

### Revendications

1. L'utilisation d'un oligopeptide d'efrapeptine dans la préparation d'un médicament pour le traitement de maladies nécessitant l'inhibition de l'angiogénèse.

2. L'emploi de la revendication no 1 où l'oligopeptide d'efrapeptine est sélectionné parmi un group comprenant SEQ ID NO:1, SEQ ID NO:2, SEQ ID NO:3, SEQ ID NO: 4, et SEQ ID NO: 5.

3. L'emploi de la revendication no 2 où une maladie liée à une angiogénèse excessive/non-contrôlée est une maladie sélectionnée parmi le groupe comprenant le cancer, l'endométriose, le sarcome de Kaposi, l'arthrite, l'athérosclérose, le psoriasis, la dégénération maculaire, la rétinopathie diabétique, le glaucome néovasculaire, l'angiogénèse du myocarde et l'obésité.

Concentration of Efrapeptin D (SEQ ID NO:2) in μM

FIG. 1

FIG. 2

FIG. 3

SEQUENCE LISTING

<110> Antonia Papathanasiou
Papathanasiou, Antonia

<120> Use of efrapeptin oligopeptides for inhibition of angiogenesis

<130> 100-02-07-13

<160> 5

<170> PatentIn version 3.1

<210> 1
<211> 15
<212> PRT
<213> Tolypocladium niveum

<220>
<221> MISC_FEATURE
<222> (1)..(1)

<223> ACETYLATION, pipecolic acid

<220>
<221> MOD_RES
<222> (2)..(2)
<223> Aib

<220>
<221> MISC_FEATURE
<222> (3)..(3)
<223> pipecolic acid

<220>
<221> MOD_RES
<222> (4)..(5)
<223> Aib

<220>
<221> MOD_RES
<222> (7)..(7)
<223> bAla

<220>
<221> MOD_RES
<222> (9)..(10)
<223> Aib

<220>
<221> MISC_FEATURE
<222> (11)..(11)
<223> pipecolic acid

<220>
<221> MOD_RES
<222> (12)..(12)
<223> Aib

<220>
<221> MOD_RES
<222> (15)..(15)
<223> BLOCKED, Aib

<400> 1

Xaa Ala Xaa Ala Ala Leu Ala Gly Ala Ala Xaa Ala Gly Leu Ala          Seq No. 1
1          5          10          15

<210> 2
<211> 15
<212> PRT
<213> Tolypocladium niveum

<220>
<221> MISC_FEATURE
<222> (1)..(1)
<223> ACETYLATION, pipecolic acid

<220>
<221> MOD_RES
<222> (2)..(2)
<223> Aib

<220>
<221> MISC_FEATURE
<22> (3)..(3)
<223> pipecolic acid

<220>
<221> MOD_RES
<222> (4)..(5)
<223> Aib

<220>
<221> MOD_RES
<222> (7)..(7)
<223> bAla

<220>
<221> MOD_RES
<222> (9)..(10)
<223> Aib

<220>
<221> MISC_FEATURE
<222> (11)..(11)
<223> pipecolic acid

<220>
<221> MOD_RES
<222> (12)..(12)
<223> Aib

<220>
<221> MOD_RES
<222> (15)..(15)
<223> BLOCKED, isovaline

<400> 2

Xaa Ala Xaa Ala Ala Leu Ala Gly Ala Ala Xaa Ala Gly Leu Val
1               5               10              15

<210> 3
<211> 15
<212> PRT
<213> Tolypolcadium niveum

<220>
<221> MISC_FEATURE
<222> (1)..(1)
<223> Acetylation, pipecolic acid

<220>

<221> MOD_RES
<222> (2)..(2)
<223> Aib

<220>
<221> MISC_FEATURE
<222> (3)..(3)
<223> pipecolic acic

<220>
<221> MOD_RES
<222> (4)..(4)
<223> isovaline

<220>
<221> MOD_RES
<222> (5)..(5)
<223> Aib

<220>
<221> MOD_RES
<222> (7)..(7)
<223> bAla

<220>
<221> MOD_RES
<222> (9)..(10)
<223> Aib

<220>
<221> MISC_FEATURE
<222> (11)..(11)
<223> pipecolic acid

<220>
<221> MOD_RES
<222> (12)..(12)
<223> Aib

<220>
<221> MOD_RES
<222> (15)..(15)
<223> BLOCKED, isovaline

<400> 3.

Xaa Ala Xaa Val Ala Leu Ala Gly Ala Ala Xaa Ala Gly Leu Val
1               5               10              15

<210> 4
<211> 15
<212> PRT
<213> Tolypocladium niveum

<220>
<221> MISC_FEATURE

<222> (1)..(1)
<223> ACETYLATION, pipecolic acid

<220>
<221> MOD_RES
<222> (2)..(2)
<223> Aib

<220>
<221> MISC_FEATURE
<222> (3)..(3)
<223> pipecolic acid

<220>
<221> MOD_RES
<222> (4)..(5)
<223> Aib

<220>
<221> MOD_RES
<222> (7)..(7)
<223> bAla

<220>
<221> MOD_RES
<222> (9)..(10)
<223> Aib

<220>
<221> MISC_FEATURE
<222> (11)..(11)
<223> pipecolic acid

<220>
<221> MOD_RES
<222> (12)..(12)
<223> Aib

<220>
<221> MOD_RES
<222> (15)..(15)
<223> BLOCKED, isovaline

<400> 4

Xaa Ala Xaa Ala Ala Leu Ala Gly Ala Ala Xaa Ala Ala Leu Val
1               5               10              15

<210> 5
<211> 15
<212> PRT
<213> Tolupocladium niveum

<220>
<221> MISC_FEATURE
<222> (1)..(1)

<223> ACETYLATION, pipecolic acid

<220>
<221> MOD_RES
<222> (2)..(2)
<223> Aib

<220>
<221> MISC_FEATURE
<222> (3)..(3)
<223> pipecolic acid

<220>
<221> MOD_RES
<222> (4)..(4)
<223> isovaline

<220>
<221> MOD_RES
<222> (5)..(5)
<223> Aib

<220>
<221> MOD_RES
<222> (7)..(7)
<223> bAla

<220>
<221> MOD_RES
<222> (9)..(10)
<223> Aib

<220>
<221> MISC_FEATURE
<222> (11)..(11)
<223> pipecolic acid

<220>
<221> MOD_RES
<222> (12)..(12)
<223> Aib

<220>
<221> MOD_RES
<222> (15)..(15)
<223> BLOCKED, isovaline

<220>
<221> MOD_RES
<222> (15)..(15)
<223> BLOCKED, isovaline

<400> 5

Xaa Ala Xaa Val Ala Leu Ala Gly Ala Ala Xaa Ala Ala Leu Val
1               5               10              15